# EUROPEAN PATENT APPLICATION

(11) **EP 4 497 447 A1**
(43) Date of publication of application: **29.01.2025**
(21) Application number: 24190753.4
(22) Date of filing: 24.07.2024
(51) Int. Cl.: A61L 2/22, A61B 1/00, A61B 90/70, B01L 1/04, F26B 9/06, F26B 21/00, F26B 21/02

(54) **METHOD AND SYSTEM FOR DRYING AND DISINFECTING ENDOSCOPES IN AN ENDOSCOPE STORAGE CABINET**

(30) Priority: 24.07.2023 IT 202300015459
(71) Applicant: Nuova S.B. System S.r.l., 20092 Cinisello Balsamo (MI) (IT)
(72) Inventor: BONGIOVANNI, Davide, 20092 Cinisello Balsamo (MI) (IT)
(74) Representative: Aprà, Mario

(57) **Abstract**

Method and system for drying and disinfecting at least one endoscope in an endoscope storage cabinet (M), wherein said cabinet (M) comprises at least one closing door and, when the cabinet (M) is closed by means of said at least one door, it provides at least one respective storage chamber (hereinafter "respective storage chamber"), in which at least one endoscope to be treated is arranged, characterized in that:
- said method comprises a first drying process and a subsequent disinfecting process of at least one endoscope to be treated in said respective storage chamber of the cabinet (M);
- said first drying process comprises at least:
- a step of blowing, from an air collection chamber (G), external and internal with respect to said cabinet (M):
- a first part of air, into said respective storage chamber of the cabinet (M), so as externally dry said at least one endoscope arranged in said respective storage chamber, and
- a second part of air, inside a manifold (L), wherein said second part of air, through a solenoid valve (L.1) for controlling air inflow and solenoid valves (L.3) for controlling air outflow, pneumatically connected with respect to corresponding internal channels of said at least one endoscope in said respective storage chamber, enters said internal channels of said at least one endoscope and dries said internal channels of said at least one endoscope arranged in said respective storage chamber;

- said subsequent disinfecting process comprises at least:
- a step of blowing, from said air collection chamber (G):
- a first part of air, with the addition of a disinfecting solution, into said respective storage chamber of the cabinet (M), so as to externally disinfect said at least one endoscope arranged in said respective storage chamber, and
- a second part of air, with the addition of a disinfecting solution, inside said manifold (L) and which, through said solenoid valve (L.1) for controlling air inflow and said solenoid valves (L.3) for controlling air outflow, enters said internal channels of said at least one endoscope and disinfects said internal channels of said at least one endoscope arranged in said respective storage chamber.

## Description

The present invention relates to a method for drying and disinfecting endoscopes in an endoscope storage cabinet.

The invention also relates to a system for drying and disinfecting endoscopes in an endoscope storage cabinet.

### Background art

With reference to the reprocessing cycle of endoscopes after use, these are washed, disinfected and/or sterilized inside automatic washer-disinfecting machines, which carry out a high level disinfection process.

At the end of the high level disinfection process, the endoscopes can be stored inside endoscope storage cabinets.

A known endoscope storage cabinet allows external drying and drying of the internal channels of endoscopes stored in the cabinet, by means of air that is suitably filtered and sterilized.

The environment inside the known endoscope storage cabinet is suitable to perform the aforesaid drying usually thanks to the presence of a positive pressure of the air inside the cabinet, which does not allow external particles and microorganisms to enter. With these features, endoscopes can be stored inside a known storage cabinet for a variable duration. There are no precise indications in the literature on how long an endoscope can effectively remain inside the storage cabinet maintaining its disinfected status: this period of time varies greatly according to different researches, which comprise a minimum time of 72 hours and a maximum time of 56 days.

The documents JP2013 070764 A, EP 1 253 951 B1, CN 203 432 233 U and CN 103 622 754 A refer to a method and/or a system for drying and disinfecting endoscopes in an endoscope storage cabinet.

### Technical problem underlying the invention

However, the prior art endoscope storage cabinet does not allow the elimination of possible microorganisms, which can enter the cabinet when it is opened, and hence does not allow the storage times of the endoscopes to be prolonged and the risk of cross contamination to be reduced.

### Summary of the invention

An object of the present invention is to provide a method for drying and disinfecting endoscopes in an endoscope storage cabinet, externally and internally to the channels of the endoscopes stored in the cabinet, which allows the elimination of possible microorganisms, which can enter the cabinet when it is opened, and hence allows the storage times of endoscopes to be prolonged and the risk of cross contamination to be reduced.

Another object of the invention is to provide a system for implementation of the aforesaid method, which is simple to produce and guarantees safe operation.

In view of these objects, the present invention provides a method for drying and disinfecting endoscopes in an endoscope storage cabinet, externally and internally to the channels of the endoscopes stored in the cabinet, the essential feature of which forms the subject matter of claim 1.

The present invention further provides a system for implementation of the method indicated above, the essential feature of which forms the subject matter of claim 5. Further advantageous features form the subject matter of the dependent claims.

### Description of the drawing

In the drawing:
- Fig. 1 is a schematic view illustrating an endoscope storage cabinet having several shelves and provided with the system for implementation of the method according to the invention in relation to the drying process of the endoscopes arranged in the cabinet;
- Fig. 2 is a schematic view illustrating the endoscope storage cabinet according to Fig. 1, provided with the system for implementation of the method according to the invention in relation to the disinfecting process of endoscopes arranged in the cabinet.

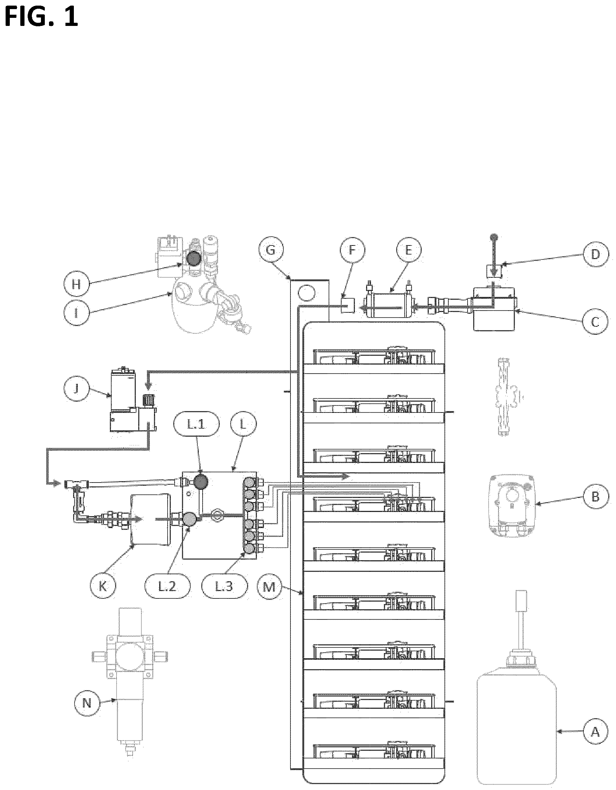

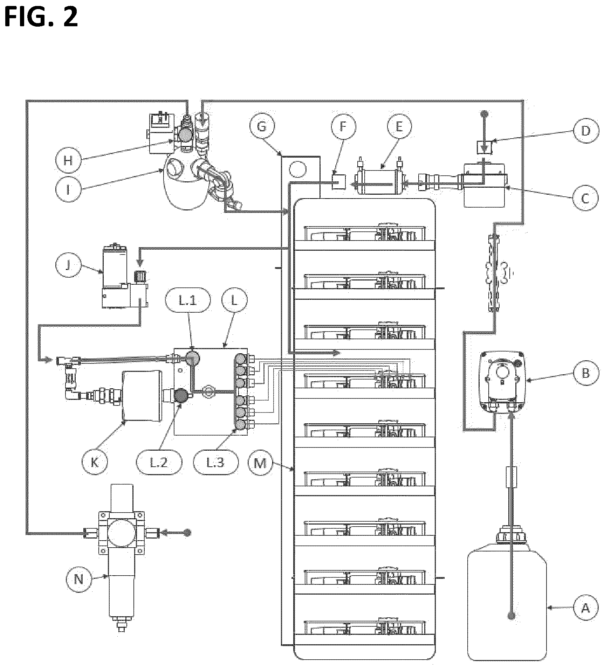

With reference to the drawing, the method according to the invention for drying and disinfecting endoscopes in an endoscope storage cabinet M, externally and internally to the channels of the endoscopes stored in the cabinet, is described. Said method comprises a drying process, described with reference to Fig. 1, and a subsequent disinfecting process, described with reference to Fig. 2.

It is specified that the endoscope storage cabinet M comprises a plurality of shelves, on each of which at least one endoscope to be treated can be placed. However, the method according to the invention can also be implemented with reference to a cabinet with a single shelf, on which at least one endoscope to be treated is placed.

In particular, said cabinet M comprises at least one closing door (known and not illustrated) and at least one shelf, which is comprised, when the cabinet M is closed by means of said at least one door, in a respective storage chamber, in which at least one endoscope to be treated is arranged.

The method according to the invention is implemented with reference to a storage chamber of the cabinet M, in which at least one endoscope to be treated is arranged. The relative system is also produced with reference to a storage chamber of the cabinet M, in which at least one endoscope to be treated is arranged.

Specifically, the invention refers to a method for drying and disinfecting at least one endoscope in an endoscope storage cabinet M, wherein said cabinet M comprises at least one closing door and, when the cabinet M is closed by means of said at least one door, it provides at least one respective storage chamber (hereinafter "respective storage chamber"), in which at least one endoscope to be treated is arranged.

Said method is characterized in that it comprises a first drying process and a subsequent disinfecting process of at least one endoscope to be treated in said respective storage chamber of the cabinet M.

In particular, said first drying process comprises at least a step of blowing, from an air collection chamber G, external and internal with respect to said cabinet M:
- a first part of air, into said respective storage chamber of the cabinet M, so as externally dry said said at least one endoscope arranged in said respective storage chamber, and
- a second part of air, inside a manifold L, wherein said second part of air, through a solenoid valve L.1 for controlling air inflow and solenoid valves L.3 for controlling air outflow of said manifold L, pneumatically connected with respect to corresponding internal channels of said at least one endoscope in said respective storage chamber, enters said internal channels of said at least one endoscope and dries said internal channels of said at least a endoscope arranged in said respective storage chamber. Moreover, said subsequent disinfecting process comprises at least a step of blowing, from said air collection chamber G:
- a first part of air, with the addition of a disinfecting solution, into said respective storage chamber of the cabinet M, so as to externally disinfect said at least one endoscope arranged in said respective storage chamber, and
- a second part of air, with the addition of a disinfecting solution, inside said manifold L and which, through said solenoid valve L.1 for controlling air inflow and said solenoid valves L.3 for controlling air outflow, enters said internal channels of said at least one endoscope and disinfects said internal channels of said at least one endoscope arranged in said respective storage chamber.

In more detail, the method according to the invention is characterized in that said first drying process comprises the steps of:
- providing a suction pump J of external air for said respective storage chamber;
- collecting, by means of said pump J, air external to the cabinet M and passing said external air through a first electrical resistor D, by means of which the external air is heated to a first temperature value T1;
- providing a blower C for said respective storage chamber;
- pushing, by means of said blower C, the external air heated to the temperature T1 through a first air filter E and passing said heated air through a second electrical resistor F, by means of which the blown air is heated further to a temperature T2 higher than T1,
- providing said air collection chamber G, external and internal with respect to said cabinet M;
- collecting the air heated further to the temperature T2 in said air collection chamber G;
- providing a nozzle for the insertion of air for said respective storage chamber and pneumatically connecting said nozzle with respect to said air collection chamber G;
- pushing a first part of the air contained in said air collection chamber G into said respective storage chamber of the cabinet M, through said nozzle, and in this way externally drying the endoscope in said respective storage chamber;
- providing a further filter K and said manifold L, having said solenoid valve for normal air inflow L.1 and said solenoid valves L.3 for outflow, corresponding to respective internal channels of said at least one endoscope in said respective storage chamber;
- pneumatically connecting said pump J to said filter K and said filter K to said solenoid valve for normal inflow L.1 of said manifold L;
- pneumatically connecting said solenoid valves L.3 for outflow from said manifold L with respect to said corresponding internal channels of said at least one endoscope into said respective storage chamber;
- pushing a second part of air from said air collection chamber G by means of said pump J through said further filter K inside said manifold L through said solenoid valve for normal inflow L.1;
- passing said second part of air through said solenoid valves L.3 for outflow from said manifold L and through said corresponding internal channels of said at least one endoscope in said respective storage chamber of the cabinet M, drying said internal channels of said at least one endoscope.

The system according to the invention for drying and disinfecting at least one endoscope in an endoscope storage cabinet M is now described, wherein said cabinet M comprises at least one closing door and, when the cabinet M is closed by means of said at least one door, it provides at least one respective storage chamber (hereinafter "respective storage chamber"), in which at least one endoscope to be treated is arranged.

Said system is characterized in that it comprises drying means and disinfecting means of at least one endoscope to be treated in said respective storage chamber of the cabinet M.

Said drying means comprise at least:
- an air collection chamber G, external and internal with respect to said cabinet M;
- a manifold L, comprising a solenoid valve L.1 for controlling air inflow and solenoid valves L.3 for controlling air outflow, which are configured to be pneumatically connected with corresponding internal channels of said at least one endoscope arranged in said respective storage chamber;
- blowing means C, provided to blow, from said air collection chamber G:
   - a first part of air, into said respective storage chamber of the cabinet M, so as to externally dry said at least one endoscope arranged in said respective storage chamber, and
   - a second part of air, inside said manifold L and which, through said solenoid valve L.1 for controlling air inflow and said solenoid valves L.3 for controlling air outflow, enters the internal channels of said at least one endoscope and dries internal channels of said at least one endoscope arranged in said respective storage chamber.

Said disinfecting means comprise at least:
- said air collection chamber G, external and internal with respect to said cabinet M,
- said manifold L, comprising said solenoid valve L.1 for controlling air inflow and said solenoid valves L.3 for controlling air outflow from said manifold L, which are pneumatically connected with corresponding internal channels of said at least one endoscope arranged in said respective storage chamber,
- said blowing means C, provided to blow, from said air collection chamber G:
   - a first part of air, with the addition of a disinfecting solution, into said respective storage chamber of the cabinet M, so as to externally disinfect said at least one endoscope arranged in said respective storage chamber, and
   - a second part of air, with the addition of a disinfecting solution, inside said manifold L and which, through said solenoid valve L.1 for controlling air inflow and said solenoid valves L.3 for controlling air outflow, enters the internal channels of said at least one endoscope and disinfects said internal channels of said at least one endoscope arranged in said respective storage chamber.

In particular, said drying means comprise:
- a suction pump J of external air provided for said respective storage chamber, which collects air external to the cabinet M and passes said external air through a first electrical resistor D, by means of which the external air is heated to a first temperature value T1;
- a blower C provided for said respective storage chamber, which pushes the air heated to the temperature T1 through a first air filter E and passes said heated air through a second electrical resistor F, by means of which the blown air is heated further to a temperature T2 higher than T1,
- said air collection chamber G, external and internal with respect to said cabinet M, in which the air heated further to the temperature T2 is collected;
- an air insertion nozzle into said respective storage chamber, which is pneumatically connected to said air collection chamber G;
   - wherein a first part of the air contained in said collection chamber G is pushed into said respective storage chamber of the cabinet M, through said nozzle, in this way externally drying said at least one endoscope in said respective storage chamber;
- a further filter K and said manifold L, comprising said solenoid valve for normal inflow L.1 and said solenoid valves L.3 for outflow, corresponding to respective internal channels of said at least one endoscope in said respective storage chamber, wherein:
   - said pump J is pneumatically connected with respect to said filter K and said filter K is pneumatically connected with respect to said solenoid valve for normal inflow L.1 of said manifold L;
   - said solenoid valves L.3 for outflow from said manifold L are pneumatically connected to corresponding internal channels of said at least one endoscope in said respective storage chamber, wherein
      - said pump J pushes a second part of air from said air collection chamber G through said further filter K into said manifold L, through said solenoid valve for normal inflow L.1 and through said solenoid valves L.3 for outflow from said manifold L, causing said second part of air to enter said corresponding internal channels of said at least one endoscope in said respective storage chamber of the cabinet M, drying the internal channels of said at least one endoscope in said respective storage chamber.

Moreover, said disinfecting means are configured to combine and atomize compressed medical air and a disinfecting solution and comprise:
- an atomizer I, which atomizes said disinfecting solution, wherein the atomized disinfecting solution is collected in said air collection chamber G;
- a blower C, which is configured to send air into said air collection chamber G, wherein by means of said blower C:
   - a first part of said atomized disinfecting solution is pushed, from said air collection chamber G, inside said respective storage chamber of the cabinet M, so as to externally disinfect said at least one endoscope arranged in said respective storage chamber;
   - a second part of said atomized disinfecting solution is pushed, from said air collection chamber G, into a pump J, which in turn pushes said atomized disinfecting solution:
      - inside said manifold L, through said solenoid valve for normal inflow L.1 and through said solenoid valves L.3 for outflow from said manifold L, which are pneumatically connected with respective internal channels of said at least one endoscope arranged in said respective storage chamber, and causes said atomized disinfecting solution to enter said internal channels of said at least one endoscope in said respective storage chamber, disinfecting them.

In particular, said disinfecting means comprise at least the following fluid circuits:
a) an external air circuit, wherein:
   - external air collected external to the cabinet M is passed through said first electrical resistor D, heating it to a first temperature T1;
   - said blower C pushes the external air into said external air circuit passing it through said first air filter E and through said second electrical resistor F, which heats it further to a second temperature T2 higher than T1, and
   - the air heated to said second temperature T2 is collected in said air collection chamber G;
b) a medical compressed air circuit, wherein:
   - medical compressed air is introduced into said atomizer I, through a solenoid valve H arranged at the inlet of said atomizer I, while the pressure of the medical compressed air is regulated by means of a pressure regulator;
   - inside said atomizer I, the medical compressed air forms a solution with a disinfecting medium;
c) a disinfecting medium circuit, wherein:
   - the disinfecting medium is drawn, by means of a suction lance, from a drum A containing said disinfecting medium, is conveyed, by means of a peristaltic pump B, to said atomizer I and enters into solution with the medical compressed air forming a disinfecting solution;
d) an atomized disinfecting solution circuit, wherein:
   - the disinfecting solution is atomized by means of said atomizer I and is collected in said air collection chamber G;
   - through the action of the air coming from said blower C:
      - a first part of the atomized disinfecting solution is pushed inside the respective storage chamber of the cabinet M, so as to externally disinfect said at least one endoscope arranged in said respective storage chamber;
      - a second part of the atomized disinfecting solution is pushed by the external air coming from said blower C into said pump J;
      - said pump J pushes the atomized disinfecting solution inside said manifold L through said solenoid valve for normal inflow L.1;
      - the disinfecting solution then passes through the solenoid valves L.3 for outflow from said manifold L, pneumatically connected with respective internal channels of said at least one endoscope arranged in said respective storage chamber, and enters said respective internal channels of said at least one endoscope, disinfecting said respective internal channels of said at least one endoscope.

### Advantages of the invention

As is apparent from the above, the invention achieves at least two particular advantages:
1. Maintaining disinfection of the endoscopes arranged in the storage cabinet, prolonging the possible duration of their storage inside the cabinet.
2. Reducing cross contamination.

It is specified that the invention does not replace the high level disinfection or sterlization that can be performed by means of endoscope washer machines, which is still necessary before being able to store the endoscopes in the storage cabinet. The invention provides a technique for maintaining high level sterilization of endoscopes that allows the guaranteed storage period in the storage cabinet to be prolonged and greatly reduces all possibility of cross contamination.

In particular, the invention allows the use of a disinfectant solution that eliminates possible microorganisms that enter the cabinet when it is opened, prolonging the storage times possible for endoscopes arranged in the cabinet and reducing the risk of cross contamination.

To summarize, according to the invention:
- the method comprises a first drying process and a subsequent disinfecting process of at least one endoscope to be treated in a respective storage chamber of the cabinet M, and
- the relative system comprises drying means and disinfecting means of at least one endoscope to be treated in said respective storage chamber of the cabinet M.

These features allow the following technical result to be achieved:
- firstly, the cabinet ensures drying of the endoscopes, both internal and external, with the use of sterile air, in particular, the internal channels of the endoscopes are connected to a closed circuit and insufflated with sterile air, which ensures that conditions of sterility are maintained;
- moreover, the external parts of the endoscopes and the storage chamber, which is subject to possible contaminations of an "extrinsic" nature (operator opening and closing the door to collect and/or deposit new endoscopes, on which the drying procedure above will be activated), are treated.

To reduce these possible sources of "extrinsic" contamination, structural and not otherwise eliminable, the system according to the invention provides for disinfection with atomization of a "dry gas" (called "dry gas system") for disinfection of the storage chamber and of the external parts of the stored endoscopes, by means of a biodegradabile disinfectant agent that is not aggressive and does not leave residues, in order to guarantee long-lasting disinfection of the chamber and of the endoscopes.

In particular, this disinfecting process is programmed at regular intervals of time, as is the distribution / reutilization of the endoscopes in their condition of internal and external sterility.

## Claims

1. Method for drying and disinfecting at least one endoscope in an endoscope storage cabinet (M), wherein said cabinet (M) comprises at least one closing door and, when the cabinet (M) is closed by means of said at least one door, it provides at least one respective storage chamber (hereinafter "respective storage chamber"), in which at least one endoscope to be treated is arranged, **characterized in that**:
- said method comprises a first drying process and a subsequent disinfecting process of at least one endoscope to be treated in said respective storage chamber of the cabinet (M);
- said first drying process comprises at least:
- a step of blowing, from an air collection chamber (G), external and internal with respect to said cabinet (M):
- a first part of air, into said respective storage chamber of the cabinet (M), so as to externally dry said at least one endoscope arranged in said respective storage chamber, and
- a second part of air, inside a manifold (L), wherein said second part of air, through a solenoid valve (L.1) for controlling air inflow and solenoid valves (L.3) for controlling air outflow, which are configured to be pneumatically connected with respect to corresponding internal channels of said at least one endoscope in said respective storage chamber, enters said internal channels of said at least one endoscope and dries said internal channels of said at least one endoscope arranged in said respective storage chamber;
- said subsequent disinfecting process comprises at least:
- a step of blowing, from said air collection chamber (G):
- a first part of air, with the addition of a disinfecting solution, into said respective storage chamber of the cabinet (M), so as to externally disinfect said at least one endoscope arranged in said respective storage chamber, and
- a second part of air, with the addition of a disinfecting solution, inside said manifold (L) and which, through said solenoid valve (L.1) for controlling air inflow and said solenoid valves (L.3) for controlling air outflow, enters said internal channels of said at least one endoscope and disinfects said internal channels of said at least one endoscope arranged in said respective storage chamber.

2. Method according to claim 1, **characterized in that** said first drying process comprises the steps of:
- providing a suction pump (J) of external air for said respective storage chamber;
- collecting, by means of said pump (J), air external to the cabinet (M) and passing said external air through a first electrical resistor (D), by means of which the external air is heated to a first temperature value (T1);
- providing a blower (C) for said respective storage chamber;
- pushing, by means of said blower (C), the external air heated to the temperature (T1) through a first air filter (E) and passing said heated air through a second electrical resistor (F), by means of which the blown air is heated further to a temperature (T2) higher than (T1),
- providing said air collection chamber (G), external and internal with respect to said cabinet (M);
- collecting the air heated further to the temperature (T2) in said air collection chamber (G);
- providing a nozzle for the insertion of air for said respective storage chamber and pneumatically connecting said nozzle with respect to said air collection chamber (G);
- pushing a first part of the air contained in said air collection chamber (G) into said respective storage chamber of the cabinet (M), through said nozzle, and in this way externally drying the endoscope in said respective storage chamber;
- providing a further filter (K) and said manifold (L), having said solenoid valve for normal air inflow (L.1) and said solenoid valves (L.3) for outflow, corresponding to respective internal channels of said at least one endoscope in said respective storage chamber;
- pneumatically connecting said pump (J) to said filter (K) and said filter (K) to said solenoid valve for normal inflow (L.1) of said manifold (L);
- pneumatically connecting said solenoid valves (L.3) for outflow from said manifold (L) to said corresponding internal channels of said at least one endoscope in said respective storage chamber;
- pushing a second part of air from said air collection chamber (G) by means of said pump (J) through said further filter (K) inside said manifold (L) through said solenoid valve for normal inflow (L.1);
- passing said second part of air through said solenoid valves (L.3) for outflow from said manifold (L) and through said corresponding internal channels of said at least one endoscope in said respective storage chamber of the cabinet (M), drying said internal channels of said at least one endoscope.

3. Method according to claim 1 and/or 2, **characterized in that** said subsequent disinfecting process is implemented utilizing a disinfecting solution and comprises at least the steps of combining and atomizing medical compressed air and said disinfecting solution, wherein:
- said disinfecting solution is atomized by means of an atomizer (I) and is collected in said air collection chamber (G), and
- by means of blown air coming from a blower (C):
- a first part of the atomized disinfecting solution is pushed inside said respective storage chamber of the cabinet (M), so as to externally disinfect the at least one endoscope arranged in said respective storage chamber;
- a second part of the disinfecting solution is pushed into a pump (J), which in turn pushes the disinfecting solution:
- into said manifold (L) and said disinfecting solution, through said solenoid valve for normal inflow (L.1) and through said solenoid valves (L.3) for outflow from said manifold (L), pneumatically connected with corresponding internal channels of said at least one endoscope arranged in said respective storage chamber, enters said internal channels of said at least one endoscope in said respective storage chamber, disinfecting them.

4. Method according to claim 3, **characterized in that** said subsequent disinfecting process comprises at least the following steps, implemented by means of respective fluid circuits:
a) an external air circuit, wherein:
- external air is collected externally to the cabinet (M), is passed through said first electrical resistor (D), heating it to the temperature (T1), and is then pushed inside said external air circuit by said blower (C), wherein the air blown by said blower (C) passes through said first air filter (E) and through said second electrical resistor (F), which heats it further to the temperature (T2) higher than (T1), and the air heated to the temperature (T2) is collected in said air collection chamber (G);
b) a medical compressed air circuit, wherein:
- medical compressed air is introduced into an atomizer (I), through a solenoid valve (H) arranged at the inlet of said atomizer (I), while the pressure of the medical compressed air is regulated by means of a pressure regulator;
- inside said atomizer (I), the medical compressed air forms a solution with a disinfecting medium;
c) a disinfecting medium circuit, wherein:
- the disinfecting medium is drawn, by means of a suction lance, from a drum (A) containing said disinfecting medium and is conveyed, by means of a peristaltic pump (B), to said atomizer (I), where it enters into solution with the medical compressed air forming a disinfecting solution;
d) an atomized disinfecting solution circuit, wherein:
- the disinfecting solution is atomized by means of said atomizer (I) and is collected in said air collection chamber (G);
- through the action of the air coming from said blower (C):
- a first part of the atomized disinfecting solution is pushed, from said air collection chamber (G), inside the respective storage chamber of the cabinet (M), so as to externally disinfect said at least one endoscope arranged in said respective storage chamber;
- a second part of the atomized disinfecting solution is pushed, from said air collection chamber (G), into said pump (J);
- said pump (J) pushes the disinfecting solution inside said manifold (L) through said solenoid valve for normal inflow (L.1);
- the disinfecting solution then passes through the solenoid valves (L.3) for outflow from said manifold (L), pneumatically connected with said corresponding internal channels of said at least one endoscope arranged in said respective storage chamber, and enters said internal channels of said at least one endoscope, disinfecting said internal channels of said at least one endoscope arranged in said respective storage chamber.

5. System for drying and disinfecting at least one endoscope in an endoscope storage cabinet (M), wherein said cabinet (M) comprises at least one closing door and, when the cabinet (M) is closed by means of said at least one door, it provides at least one respective storage chamber (hereinafter "respective storage chamber"), in which at least one endoscope to be treated is arranged, **characterized in that** said system comprises drying means and disinfecting means of at least one endoscope to be treated in said respective storage chamber of the cabinet (M), and **in that**:
- said drying means comprise at least:
- an air collection chamber (G), external and internal with respect to said cabinet (M);
- a manifold (L), comprising a solenoid valve (L.1) for controlling air inflow and solenoid valves (L.3) for controlling air outflow, which are configured to be pneumatically connected with corresponding internal channels of said at least one endoscope arranged in said respective storage chamber;
- blowing means (C), wherein said blowing means are provided to blow, from said air collection chamber (G):
- a first part of air into said respective storage chamber of the cabinet (M), so as to externally dry said at least one endoscope arranged in said respective storage chamber, and
- a second part of air, inside said manifold (L) and which, through said solenoid valve (L.1) for controlling air inflow and said solenoid valves (L.3) for controlling air outflow, enters the internal channels of said at least one endoscope and dries the internal channels of said at least one endoscope arranged in said respective storage chamber;
- said disinfecting means comprise at least:
- said air collection chamber (G), external and internal with respect to said cabinet (M),
- said manifold (L), comprising said solenoid valve (L.1) for controlling air inflow and said solenoid valves (L.3) for controlling air outflow from said manifold (L), which are pneumatically connected with corresponding internal channels of said at least one endoscope arranged in said respective storage chamber,
- said blowing means (C), wherein said blowing means are provided to blow, from said air collection chamber (G):
- a first part of air, with the addition of a disinfecting solution, into said respective storage chamber of the cabinet (M), so as to externally disinfect said at least one endoscope arranged in said respective storage chamber, and
- a second part of air, with the addition of a disinfecting solution, inside said manifold (L) and which, through said solenoid valve (L.1) for controlling air inflow and said solenoid valves (L.3) for controlling air outflow, enters the internal channels of said at least one endoscope and disinfects said internal channels of said at least one endoscope arranged in said respective storage chamber.

6. System according to claim 5, **characterized in that** said drying means comprise:
- a suction pump (J) of external air provided for said respective storage chamber, which collects air external to the cabinet (M) and passes said external air through a first electrical resistor (D), by means of which the external air is heated to a first temperature value (T1);
- a blower (C) provided for said respective storage chamber, which pushes the air heated to the temperature (T1) through a first air filter (E) and passes said heated air through a second electrical resistor (F), by means of which the blown air is heated further to a temperature (T2) higher than (T1),
- said air collection chamber (G), external and internal with respect to said cabinet (M), in which the air heated further to the temperature (T2) is collected;
- an air insertion nozzle into said respective storage chamber, which is pneumatically connected to said air collection chamber (G);
- wherein a first part of the air contained in said collection chamber (G) is pushed into said respective storage chamber of the cabinet (M), through said nozzle, in this way externally drying said at least one endoscope in said respective storage chamber;
- a further filter (K) and said manifold (L), comprising said solenoid valve for normal inflow (L.1) and said solenoid valves (L.3) for outflow, corresponding to respective internal channels of said at least one endoscope in said respective storage chamber, wherein:
- said pump (J) is pneumatically connected with respect to said filter (K) and said filter (K) is pneumatically connected with respect to said solenoid valve for normal inflow (L.1) of said manifold (L);
- said solenoid valves (L.3) for outflow from said manifold (L) are pneumatically connected to corresponding internal channels of said at least one endoscope in said respective storage chamber, wherein:
- said pump (J) pushes a second part of air from said air collection chamber (G) through said further filter (K) into said manifold (L), through said solenoid valve for normal inflow (L.1) and through said solenoid valves (L.3) for outflow from said manifold (L), causing said second part of air to enter said corresponding internal channels of said at least one endoscope in said respective storage chamber of the cabinet (M), drying the internal channels of said at least one endoscope in said respective storage chamber.

7. System according to claim 5, **characterized in that** said disinfecting means are configured to combine and atomize compressed medical air and a disinfecting solution and comprise:
- an atomizer (I), which atomizes said disinfecting solution, wherein the atomized disinfecting solution is collected in said air collection chamber (G);
- a blower (C), which is configured to send air into said air collection chamber (G), wherein by means of said blower (C):
- a first part of said atomized disinfecting solution is pushed, from said air collection chamber (G), inside said respective storage chamber of the cabinet (M), so as to externally disinfect said at least one endoscope arranged in said respective storage chamber;
- a second part of said atomized disinfecting solution is pushed, from said air collection chamber (G), into a pump (J), which in turn pushes said atomized disinfecting solution:
- inside said manifold (L), through said solenoid valve for normal inflow (L.1) and through said solenoid valves (L.3) for outflow from said manifold (L), which are pneumatically connected with respective internal channels of said at least one endoscope arranged in said respective storage chamber, and causes said atomized disinfecting solution to enter said internal channels of said at least one endoscope in said respective storage chamber, disinfecting them.

8. System according to claim 6, **characterized in that** said disinfecting means comprise at least the following fluid circuits:
a) an external air circuit, wherein:
- external air collected external to the cabinet (M) is passed through said first electrical resistor (D), heating it to a first temperature (T1);
- said blower (C) pushes the external air into said external air circuit passing it through said first air filter (E) and through said second electrical resistor (F), which heats it further to a second temperature (T2) higher than (T1), and
- the air heated to said second temperature (T2) is collected in said air collection chamber (G);
b) a medical compressed air circuit, wherein:
- medical compressed air is introduced into an atomizer (I), through a solenoid valve (H) arranged at the inlet of said atomizer (I), while the pressure of the medical compressed air is regulated by means of a pressure regulator;
- inside said atomizer (I), the medical compressed air forms a solution with a disinfecting medium;
c) a disinfecting medium circuit, wherein:
- the disinfecting medium is drawn, by means of a suction lance, from a drum (A) containing said disinfecting medium, is conveyed, by means of a peristaltic pump (B), to said atomizer (I) and enters into solution with the medical compressed air forming a disinfecting solution;
d) an atomized disinfecting solution circuit, wherein:
- the disinfecting solution is atomized by means of said atomizer (I) and is collected in said air collection chamber (G);
- through the action of the air coming from said blower (C):
- a first part of the atomized disinfecting solution is pushed inside the respective storage chamber of the cabinet (M), so as to externally disinfect said at least one endoscope arranged in said respective storage chamber;
- a second part of the atomized disinfecting solution is pushed by the external air coming from said blower (C) into said pump (J);
- said pump (J) pushes the atomized disinfecting solution inside said manifold (L) through said solenoid valve for normal inflow (L.1);
- the disinfecting solution then passes through the solenoid valves (L.3) for outflow from said manifold (L), pneumatically connected with respective internal channels of said at least one endoscope arranged in said respective storage chamber, and enters said respective internal channels of said at least one endoscope, disinfecting said respective internal channels of said at least one endoscope.
